# EUROPÄISCHE PATENTSCHRIFT

(11) **EP 0 775 203 B1**
(45) Veröffentlichungstag und Bekanntmachung des Hinweises auf die Patenterteilung: **12.11.2003**
(21) Anmeldenummer: 95929028.9
(22) Anmeldetag: 31.07.1995
(51) Int. Cl.: C12N 15/10, C12N 15/63, C12N 15/65, C12N 15/85, A61K 48/00

(54) **GENTHERAPEUTISCHES VERFAHREN UNTER VERWENDUNG VON DNA-VEKTOREN OHNE SELEKTIONSMARKER-GEN**
METHOD OF GENE THERAPY USING DNA VECTORS WITHOUT SELECTION MARKER GENE
PROCEDE DE THERAPIE GENIQUE UTILISANT DES VECTEURS D'ADN SANS GENE MARQUEUR DE SELECTION

(30) Priorität: 11.08.1994 DE 4428402
(43) Veröffentlichungstag der Anmeldung: 28.05.1997
(73) Patentinhaber: Roche Diagnostics GmbH, 68305 Mannheim (DE)
(72) Erfinder: SEEBER, Stefan, D-82377 Penzberg (DE); RÜGER, Rüdiger, D-82386 Huglfing (DE)
(86) Internationale Anmeldenummer: EP9503027
(87) Internationale Veröffentlichungsnummer: WO96005297

(56) Entgegenhaltungen:
- WO-A-94/09127
- WO-A-95/25800
- PROC. NATL.ACAD SCI., Bd. 90, Januar 1993 NATL. ACAD SCI.,WASHINGTON,DC,US;, Seiten 472-476, H.E. CHOY AND S.ADHYA 'RNA polymerase idling and clearance in gal promoters: Use of supercoiled minicircle DNA template made in vivo'
- J. BIOL. CHEM., Bd. 269, Nr. 18, 6.Mai 1994 AM. SOC. BIOCHEM. MOL.BIOL.,INC.,BALTIMORE,US, Seiten 13511-13521, T.T. SU AND W.R.MCCLURE 'Selective binding of Escherichia coli RNA polymerase to Topoisomers of minicircles carrying the TAC16 and TAC17 promoters'
- GENE, Bd. 70, 1988 ELSEVIER SCIENCE PUBLISHERS,B.V.,AMSTERDAM,NL;, Seiten 331-341, B. SAUER AND N. HERNDERSON 'The cyclization of linear DNA in Escherichia coli by site-specific recombination' in der Anmeldung erwähnt
- NATURE, Bd. 362, 18.März 1993 MACMILLAN JOURNALS LTD., LONDON,UK, Seiten 250-255, S.C. HYDE ET AL. 'Correction of the ion transport defect in cystic fibrosis transgenic mice by gene therapy' in der Anmeldung erwähnt

### SEQUENZPROTOKOLL

(1) ALLGEMEINE ANGABEN:
   (i) ANMELDER:
      (A) NAME: BOEHRINGER MANNHEIM GMBH
      (B) STRASSE: Sandhofer Str. 116
      (C) ORT: Mannheim
      (E) LAND: Deutschland
      (F) POSTLEITZAHL: D-68305
      (G) TELEFON: 08856/60-3446
      (H) TELEFAX: 08856/60-3451
   (ii) BEZEICHNUNG DER ERFINDUNG: Gentherapeutisches Verfahren unter Verwendung von DNA-Vektoren ohne Selektionsmarker-Gen
   (iii) ANZAHL DER SEQUENZEN: 5
   (iv) COMPUTER-LESBARE FASSUNG:
      (A) DATENTRÄGER: Floppy disk
      (B) COMPUTER: IBM PC compatible
      (C) BETRIEBSSYSTEM: PC-DOS/MS-DOS
      (D) SOFTWARE: PatentIn Release #1.0, Version #1.30B (EPA)
   (vi) DATEN DER URANMELDUNG:
      (A) ANMELDENUMMER: DE P 44 28 402.0
      (B) ANMELDETAG: 11-AUG-1994
(2) ANGABEN ZU SEQ ID NO: 1:
   (i) SEQUENZKENNZEICHEN:
      (A) LÄNGE: 8225 Basenpaare
      (B) ART: Nucleotid
      (C) STRANGFORM: Doppelstrang
      (D) TOPOLOGIE: linear
   (ii) ART DES MOLEKÜLS: cDNA
   (xi) SEQUENZBESCHREIBUNG: SEQ ID NO: 1:
   GGCCGCCGCG GCCTCGAGGA GCTTGGCCCA TTGCATACGT TGTATCCATA TCATAATATG 60
   TACATTTATA TTGGCTCATG TCCAACATTA CCGCCATGTT GACATTGATT ATTGACTAGT 120
   TATTAATAGT AATCAATTAC GGGGTCATTA GTTCATAGCC CATATATGGA GTTCCGCGTT 180
   ACATAACTTA CGGTAAATGG CCCGCCTGGC TGACCGCCCA ACGACCCCCG CCCATTGACG 240
   TCAATAATGA CGTATGTTCC CATAGTAACG CCAATAGGGA CTTTCCATTG ACGTCAATGG 300
   GTGGAGTATT TACGGTAAAC TGCCCACTTG GCAGTACATC AAGTGTATCA TATGCCAAGT 360
   ACGCCCCCTA TTGACGTCAA TGACGGTAAA TGGCCCGCCT GGCATTATGC CCAGTACATG 420
   ACCTTATGGG ACTTTCCTAC TTGGCAGTAC ATCTACGTAT TAGTCATCGC TATTACCATG 480
   GTGATGCGGT TTTGGCAGTA CATCAATGGG CGTGGATAGC GGTTTGACTC ACGGGGATTT 540
   CCAAGTCTCC ACCCCATTGA CGTCAATGGG AGTTTGTTTT GGCACCAAAA TCAACGGGAC 600
   TTTCCAAAAT GTCGTAACAA CTCCGCCCCA TTGACGCAAA TGGGCGGTAG GCGTGTACGG 660
   TGGGAGGTCT ATATAAGCAG AGCTCGTTTA GTGAACCGTC AGATCGCCTG GAGACGCCAT 720
   CCACGCTGTT TTGACCTCCA TAGAAGACAC CGGGACCGAT CCAGCCTCCC CGGCTAGGGT 780
   CGACGGTACC CGAGAGACCA TGCAGAGGTC GCCTCTGGAA AAGGCCAGCG TTGTCTCCAA 840
   ACTTTTTTTC AGCTGGACCA GACCAATTTT GAGGAAAGGA TACAGACAGC GCCTGGAATT 900
   GTCAGACATA TACCAAATCC CTTCTGTTGA TTCTGCTGAC AATCTATCTG AAAAATTGGA 960
   AAGAGAATGG GATAGAGAGC TGGCTTCAAA GAAAAATCCT AAACTCATTA ATGCCCTTCG 1020
   GCGATGTTTT TTCTGGAGAT TTATGTTCTA TGGAATCTTT TTATATTTAG GGGAAGTCAC 1080
   CAAAGCAGTA CAGCCTCTCT TACTGGGAAG AATCATAGCT TCCTATGACC CGGATAACAA 1140
   GGAGGAACGC TCTATCGCGA TTTATCTAGG CATAGGCTTA TGCCTTCTCT TTATTGTGAG 1200
   GACACTGCTC CTACACCCAG CCATTTTTGG CCTTCATCAC ATTGGAATGC AGATGAGAAT 1260
   AGCTATGTTT AGTTTGATTT ATAAGAAGAC TTTAAAGCTG TCAAGCCGTG TTCTAGATAA 1320
   AATAAGTATT GGACAACTTG TTAGTCTCCT TTCCAACAAC CTGAACAAAT TTGATGAAGG 1380
   ACTTGCATTG GCACATTTCG TGTGGATCGC TCCTTTGCAA GTGGCACTCC TCATGGGGCT 1440
   AATCTGGGAG TTGTTACAGG CGTCTGCCTT CTGTGGACTT GGTTTCCTGA TAGTCCTTGC 1500
   CCTTTTTCAG GCTGGGCTAG GGAGAATGAT GATGAAGTAC AGAGATCAGA GAGCTGGGAA 1560
   GATCAGTGAA AGACTTGTGA TTACCTCAGA AATGATTGAA AACATCCAAT CTGTTAAGGC 1620
   ATACTGCTGG GAAGAAGCAA TGGAAAAAAT GATTGAAAAC TTAAGACAAA CAGAACTGAA 1680
   ACTGACTCGG AAGGCAGCCT ATGTGAGATA CTTCAATAGC TCAGCCTTCT TCTTCTCAGG 1740
   GTTCTTTGTG GTGTTTTTAT CTGTGCTTCC CTATGCACTA ATCAAAGGAA TCATCCTCCG 1800
   GAAAATATTC ACCACCATCT CATTCTGCAT TGTTCTGCGC ATGGCGGTCA CTCGGCAATT 1860
   TCCCTGGGCT GTACAAACAT GGTATGACTC TCTTGGAGCA ATAAACAAAA TACAGGATTT 1920
   CTTACAAAAG CAAGAATATA AGACATTGGA ATATAACTTA ACGACTACAG AAGTAGTGAT 1980
   GGAGAATGTA ACAGCCTTCT GGGAGGAGGG ATTTGGGGAA TTATTTGAGA AAGCAAAACA 2040
   AAACAATAAC AATAGAAAAA CTTCTAATGG TGATGACAGC CTCTTCTTCA GTAATTTCTC 2100
   ACTTCTTGGT ACTCCTGTCC TGAAAGATAT TAATTTCAAG ATAGAAAGAG GACAGTTGTT 2160
   GGCGGTTGCT GGATCCACTG GAGCAGGCAA GACTTCACTT CTAATGATGA TTATGGGAGA 2220
   ACTGGAGCCT TCAGAGGGTA AAATTAAGCA CAGTGGAAGA ATTTCATTCT GTTCTCAGTT 2280
   TTCCTGGATT ATGCCTGGCA CCATTAAAGA AAATATCATC TTTGGTGTTT CCTATGATGA 2340
   ATATAGATAC AGAAGCGTCA TCAAAGCATG CCAACTAGAA GAGGACATCT CCAAGTTTGC 2400
   AGAGAAAGAC AATATAGTTC TTGGAGAAGG TGGAATCACA CTGAGTGGAG GTCAACGAGC 2460
   AAGAATTTCT TTAGCAAGAG CAGTATACAA AGATGCTGAT TTGTATTTAT TAGACTCTCC 2520
   TTTTGGATAC CTAGATGTTT TAACAGAAAA AGAAATATTT GAAAGCTGTG TCTGTAAACT 2580
   GATGGCTAAC AAAACTAGGA TTTTGGTCAC TTCTAAAATG GAACATTTAA AGAAAGCTGA 2640
   CAAAATATTA ATTTTGCATG AAGGTAGCAG CTATTTTTAT GGGACATTTT CAGAACTCCA 2700
   AAATCTACAG CCAGACTTTA GCTCAAAACT CATGGGATGT GATTCTTTCG ACCAATTTAG 2760
   TGCAGAAAGA AGAAATTCAA TCCTAACTGA GACCTTACAC CGTTTCTCAT TAGAAGGAGA 2820
   TGCTCCTGTC TCCTGGACAG AAACAAAAAA ACAATCTTTT AAACAGACTG GAGAGTTTGG 2880
   GGAAAAAAGG AAGAATTCTA TTCTCAATCC AATCAACTCT ATACGAAAAT TTTCCATTGT 2940
   GCAAAAGACT CCCTTACAAA TGAATGGCAT CGAAGAGGAT TCTGATGAGC CTTTAGAGAG 3000
   AAGGCTGTCC TTAGTACCAG ATTCTGAGCA GGGAGAGGCG ATACTGCCTC GCATCAGCGT 3060
   GATCAGCACT GGCCCCACGC TTCAGGCACG AAGGAGGCAG TCTGTCCTGA ACCTGATGAC 3120
   ACACTCAGTT AACCAAGGTC AGAACATTCA CCGAAAGACA ACAGCATCCA CACGAAAAGT 3180
   GTCACTGGCC CCTCAGGCAA ACTTGACTGA ACTGGATATA TATTCAAGAA GGTTATCTCA 3240
   AGAAACTGGC TTGGAAATAA GTGAAGAAAT TAACGAAGAA GACTTAAAGG AGTGCCTTTT 3300
   TGATGATATG GAGAGCATAC CAGCAGTGAC TACATGGAAC ACATACCTTC GATATATTAC 3360
   TGTCCACAAG AGCTTAATTT TTGTGCTAAT TTGGTGCTTA GTAATTTTTC TGGCAGAGGT 3420
   GGCTGCTTCT TTGGTTGTGC TGTGGCTCCT TGGAAACACT CCTCTTCAAG ACAAAGGGAA 3480
   TAGTACTCAT AGTAGAAATA ACAGCTATGC AGTGATTATC ACCAGCACCA GTTCGTATTA 3540
   TGTGTTTTAC ATTTACGTGG GAGTAGCCGA CACTTTGCTT GCTATGGGAT TCTTCAGAGG 3600
   TCTACCACTG GTGCATACTC TAATCACAGT GTCGAAAATT TTACACCACA AAATGTTACA 3660
   TTCTGTTCTT CAAGCACCTA TGTCAACCCT CAACACGTTG AAAGCAGGTG GGATTCTTAA 3720
   TAGATTCTCC AAAGATATAG CAATTTTGGA TGACCTTCTG CCTCTTACCA TATTTGACTT 3780
   CATCCAGTTG TTATTAATTG TGATTGGAGC TATAGCAGTT GTCGCAGTTT TACAACCCTA 3840
   CATCTTTGTT GCAACAGTGC CAGTGATAGT GGCTTTTATT ATGTTGAGAG CATATTTCCT 3900
   CCAAACCTCA CAGCAACTCA AACAACTGGA ATCTGAAGGC AGGAGTCCAA TTTTCACTCA 3960
   TCTTGTTACA AGCTTAAAAG GACTATGGAC ACTTCGTGCC TTCGGACGGC AGCCTTACTT 4020
   TGAAACTCTG TTCCACAAAG CTCTGAATTT ACATACTGCC AACTGGTTCT TGTACCTGTC 4080
   AACACTGCGC TGGTTCCAAA TGAGAATAGA AATGATTTTT GTCATCTTCT TCATTGCTGT 4140
   TACCTTCATT TCCATTTTAA CAACAGGAGA AGGAGAAGGA AGAGTTGGTA TTATCCTGAC 4200
   TTTAGCCATG AATATCATGA GTACATTGCA GTGGGCTGTA AACTCCAGCA TAGATGTGGA 4260
   TAGCTTGATG CGATCTGTGA GCCGAGTCTT TAAGTTCATT GACATGCCAA CAGAAGGTAA 4320
   ACCTACCAAG TCAACCAAAC CATACAAGAA TGGCCAACTC TCGAAAGTTA TGATTATTGA 4380
   GAATTCACAC GTGAAGAAAG ATGACATCTG GCCCTCAGGG GGCCAAATGA CTGTCAAAGA 4440
   TCTCACAGCA AAATACACAG AAGGTGGAAA TGCCATATTA GAGAACATTT CCTTCTCAAT 4500
   AAGTCCTGGC CAGAGGGTGG GCCTCTTGGG AAGAACTGGA TCAGGGAAGA GTACTTTGTT 4560
   ATCAGCTTTT TTGAGACTAC TGAACACTGA AGGAGAAATC CAGATCGATG GTGTGTCTTG 4620
   GGATTCAATA ACTTTGCAAC AGTGGAGGAA AGCCTTTGGA GTGATACCAC AGAAAGTATT 4680
   TATTTTTTCT GGAACATTTA GAAAAAACTT GGATCCCTAT GAACAGTGGA GTGATCAAGA 4740
   AATATGGAAA GTTGCAGATG AGGTTGGGCT CAGATCTGTG ATAGAACAGT TTCCTGGGAA 4800
   GCTTGACTTT GTCCTTGTGG ATGGGGGCTG TGTCCTAAGC CATGGCCACA AGCAGTTGAT 4860
   GTGCTTGGCT AGATCTGTTC TCAGTAAGGC GAAGATCTTG CTGCTTGATG AACCCAGTGC 4920
   TCATTTGGAT CCAGTAACAT ACCAAATAAT TAGAAGAACT CTAAAACAAG CATTTGCTGA 4980
   TTGCACAGTA ATTCTCTGTG AACACAGGAT AGAAGCAATG CTGGAATGCC AACAATTTTT 5040
   GGTCATAGAA GAGAACAAAG TGCGGCAGTA CGATTCCATC CAGAAACTGC TGAACGAGAG 5100
   GAGCCTCTTC CGGCAAGCCA TCAGCCCCTC CGACAGGGTG AAGCTCTTTC CCCACCGGAA 5160
   CTCAAGCAAG TGCAAGTCTA AGCCCCAGAT TGCTGCTCTG AAAGAGGAGA CAGAAGAAGA 5220
   GGTGCAAGAT ACAAGGCTTT AGAGAGCAGC ATAAATGTTG ACATGGGACA TTTGCTCATG 5280
   GAATTGGAGC TCGTCGACTC TAGAGGATCC CCGGGCGAGC TCGAATTCAA GCTTGGGATC 5340
   TTTGTGAAGG AACCTTACTT CTGTGGTGTG ACATAATTGG ACAAACTACC TACAGAGATT 5400
   TAAAGCTCTA AGGTAAATAT AAAATTTTTA AGTGTATAAT GTGTTAAACT ACTGATTCTA 5460
   ATTGTTTGTG TATTTTAGAT TCACAGTCCC AAGGCTCATT TCAGGCCCCT CAGTCCTCAC 5520
   AGTCTGTTCA TGATCATAAT CAGCCATACC ACATTTGTAG AGGTTTTACT TGCTTTAAAA 5580
   AACCTCCCAC ACCTCCCCCT GAACCTGAAA CATAAAATGA ATGCAATTGT TGTTGTTAAC 5640
   TTGTTTATTG CAGCTTATAA TGGTTACAAA TAAAGCAATA GCATCACAAA TTTCACAAAT 5700
   AAAGCATTTT TTTCACTGCA TTCTAGTTGT GGTTTGTCCA AACTCATCAA TGTATCTTAT 5760
   CATGTCTGGA TCCCCGGGTA CCGAGCTCGA ATTAGCGGCC GCTAATTCGT AATCATGGTC 5820
   ATAGCTGTTT CCTGTGTGAA ATTGTTATCC GCTCACAATT CCACACAACA TACGAGCCGG 5880
   AAGCATAAAG TGTAAAGCCT GGGGTGCCTA ATGAGTGAGC TAACTCACAT TAATTGCGTT 5940
   GCGCTCACTG CCCGCTTTCC AGTCGGGAAA CCTGTCGTGC CAGCTGCATT AATGAATCGG 6000
   CCAACGCGCG GGGAGAGGCG GTTTGCGTAT TGGGCGCTCT TCCGCTTCCT CGCTCACTGA 6060
   CTCGCTGCGC TCGGTCGTTC GGCTGCGGCG AGCGGTATCA GCTCACTCAA AGGCGGTAAT 6120
   ACGGTTATCC ACAGAATCAG GGGATAACGC AGGAAAGAAC ATGTGAGCAA AAGGCCAGCA 6180
   AAAGGCCAGG AACCGTAAAA AGGCCGCGTT GCTGGCGTTT TTCCATAGGC TCCGCCCCCC 6240
   TGACGAGCAT CACAAAAATC GACGCTCAAG TCAGAGGTGG CGAAACCCGA CAGGACTATA 6300
   AAGATACCAG GCGTTTCCCC CTGGAAGCTC CCTCGTGCGC TCTCCTGTTC CGACCCTGCC 6360
   GCTTACCGGA TACCTGTCCG CCTTTCTCCC TTCGGGAAGC GTGGCGCTTT CTCATAGCTC 6420
   ACGCTGTAGG TATCTCAGTT CGGTGTAGGT CGTTCGCTCC AAGCTGGGCT GTGTGCACGA 6480
   ACCCCCCGTT CAGCCCGACC GCTGCGCCTT ATCCGGTAAC TATCGTCTTG AGTCCAACCC 6540
   GGTAAGACAC GACTTATCGC CACTGGCAGC AGCCACTGGT AACAGGATTA GCAGAGCGAG 6600
   GTATGTAGGC GGTGCTACAG AGTTCTTGAA GTGGTGGCCT AACTACGGCT ACACTAGAAG 6660
   GACAGTATTT GGTATCTGCG CTCTGCTGAA GCCAGTTACC TTCGGAAAAA GAGTTGGTAG 6720
   CTCTTGATCC GGCAAACAAA CCACCGCTGG TAGCGGTGGT TTTTTTGTTT GCAAGCAGCA 6780
   GATTACGCGC AGAAAAAAAG GATCTCAAGA AGATCCTTTG ATCTTTTCTA CGGGGTCTGA 6840
   CGCTCAGTGG AACGAAAACT CACGTTAAGG GATTTTGGTC ATGAGATTAT CAAAAAGGAT    6900
   CTTCACCTAG ATCCTTTTAA ATTAAAAATG AAGTTTTAAA TCAATCTAAA GTATATATGA    6960
   GTAAACTTGG TCTGACAGTT ACCAATGCTT AATCAGTGAG GCACCTATCT CAGCGATCTG    7020
   TCTATTTCGT TCATCCATAG TTGCCTGACT CCCCGTCGTG TAGATAACTA CGATACGGGA    7080
   GGGCTTACCA TCTGGCCCCA GTGCTGCAAT GATACCGCGA GACCCACGCT CACCGGCTCC    7140
   AGATTTATCA GCAATAAACC AGCCAGCCGG AAGGGCCGAG CGCAGAAGTG GTCCTGCAAC    7200
   TTTATCCGCC TCCATCCAGT CTATTAATTG TTGCCGGGAA GCTAGAGTAA GTAGTTCGCC    7260
   AGTTAATAGT TTGCGCAACG TTGTTGCCAT TGCTACAGGC ATCGTGGTGT CACGCTCGTC    7320
   GTTTGGTATG GCTTCATTCA GCTCCGGTTC CCAACGATCA AGGCGAGTTA CATGATCCCC    7380
   CATGTTGTGC AAAAAAGCGG TTAGCTCCTT CGGTCCTCCG ATCGTTGTCA GAAGTAAGTT    7440
   GGCCGCAGTG TTATCACTCA TGGTTATGGC AGCACTGCAT AATTCTCTTA CTGTCATGCC    7500
   ATCCGTAAGA TGCTTTTCTG TGACTGGTGA GTACTCAACC AAGTCATTCT GAGAATAGTG    7560
   TATGCGGCGA CCGAGTTGCT CTTGCCCGGC GTCAATACGG GATAATACCG CGCCACATAG    7620
   CAGAACTTTA AAAGTGCTCA TCATTGGAAA ACGTTCTTCG GGGCGAAAAC TCTCAAGGAT    7680
   CTTACCGCTG TTGAGATCCA GTTCGATGTA ACCCACTCGT GCACCCAACT GATCTTCAGC    7740
   ATCTTTTACT TTCACCAGCG TTTCTGGGTG AGCAAAAACA GGAAGGCAAA ATGCCGCAAA    7800
   AAAGGGAATA AGGGCGACAC GGAAATGTTG AATACTCATA CTCTTCCTTT TTCAATATTA    7860
   TTGAAGCATT TATCAGGGTT ATTGTCTCAT GAGCGGATAC ATATTTGAAT GTATTTAGAA    7920
   AAATAAACAA ATAGGGGTTC CGCGCACATT TCCCCGAAAA GTGCCACCTG ACGTCTAAGA    7980
   AACCATTATT ATCATGACAT TAACCTATAA AAATAGGCGT ATCACGAGGC CCTTTCGTCT    8040
   CGCGCGTTTC GGTGATGACG GTGAAAACCT CTGACACATG CAGCTCCCGG AGACGGTCAC    8100
   AGCTTGTCTG TAAGCGGATG CCGGGAGCAG ACAAGCCCGT CAGGGCGCGT CAGCGGGTGT    8160
   TGGCGGGTGT CGGGGCTGGC TTAACTATGC GGCATCAGAG CAGATTGTAC TGAGAGTGCA    8220
   CCATA                                                               8225
(2) ANGABEN ZU SEQ ID NO: 2:
   (i) SEQUENZKENNZEICHEN:
      (A) LÄNGE: 18 Basenpaare
      (B) ART: Nucleotid
      (C) STRANGFORM: Doppelstrang
      (D) TOPOLOGIE: linear
   (ii) ART DES MOLEKÜLS: cDNA
   (xi) SEQUENZBESCHREIBUNG: SEQ ID NO: 2:
   GCTGCAGCGA TCGCACGT 18
(2) ANGABEN ZU SEQ ID NO: 3:
   (i) SEQUENZKENNZEICHEN:
      (A) LANGE: 17 Basenpaare
      (B) ART: Nucleotid
      (C) STRANGFORM: Doppelstrang
      (D) TOPOLOGIE: linear
   (ii) ART DES MOLEKÜLS: cDNA
   (xi) SEQUENZBESCHREIBUNG: SEQ ID NO: 3:
   CATGCTGCAG CGATCGC 17
(2) ANGABEN ZU SEQ ID NO: 4:
   (i) SEQUENZKENNZEICHEN:
      (A) LÄNGE: 16 Basenpaare
      (B) ART: Nucleotid
      (C) STRANGFORM: Doppelstrang
      (D) TOPOLOGIE: linear
   (ii) ART DES MOLEKÜLS: cDNA
   (xi) SEQUENZBESCHREIBUNG: SEQ ID NO: 4:
   GCGATCGCTG CAGCCA 16
(2) ANGABEN ZU SEQ ID NO: 5:
   (i) SEQUENZKENNZEICHEN:
      (A) LÄNGE: 60 Basenpaare
      (B) ART: Nucleotid
      (C) STRANGFORM: Doppelstrang
      (D) TOPOLOGIE: linear
   (ii) ART DES MOLEKÜLS: cDNA
   (Xi) SEQUENZBESCHREIBUNG: SEQ ID NO: 5:
   AGCTTCGATC GCTGCAGTCG ACTCTAGAGG ATCCCGGGCG GCCGCTGCAG CGATCGAATT 60

## Beschreibung

Die Erfindung betrifft die Verwendung von Vektor-DNA ohne Selektionsmarker-Gen in der Gentherapie sowie die Verwendung dieser Vektoren zur Herstellung von Arzneimitteln für die Gentherapie.

Gentherapie von somatischen Zellen kann beispielsweise unter Verwendung von retroviralen Vektoren, anderen viralen Vektoren oder durch nichtviralen Gentransfer erfolgen (zur Übersicht vgl. T. Friedmann (1989) *(1)*, Morgan (1993) *(2)*)

Gentherapeutisch geeignete Deliverysysteme sind beispielsweise Retroviren (Mulligan, R.C. (1991) *(3)*), Adeno associated virus (McLughlin (1988) *(4)*), Vaccina virus, (Moss et al. (1987) *(5)*), Bovine papiloma virus, (Rasmussen et al. (1987) *(6)*) oder Viren aus der Gruppe der Herpesviren, wie Eppstein Barr virus (Margolskee et al. (1988) *(7)*) oder Herpes Simplex virus.

Es sind auch nichtvirale Deliverysysteme bekannt. Hierzu wird üblicherweise "nackte" Nukleinsäure, vorzugsweise DNA, verwendet, oder Nukleinsäure zusammen mit einem Hilfsstoff, wie z. B. mit Transferreagenzien (Liposomen, Dendromere, Polylysin-Transferrin-Konjugate (Wagner et al. (1990) *(14),* (Felgner et al. (1987) *(8)*).

Um die für die Gentherapie verwendbare Nukleinsäure in einer therapeutischen Menge zur Verfügung zu haben, müssen diese Nukleinsäuren vor der therapeutischen Anwendung vermehrt werden. Dazu erfolgt mindestens ein Selektionsschritt, der sich ein auf der Nukleinsäure befindliches Markergen und dessen Genprodukt zunutze macht. Übliche Selektionsmarker sind beispielsweise Ampicillin, Chloramphenicol, Erythromycin, Kanamycin, Neomycin und Tetracylin (Davies et al. (1978) *(9)*).

Es sind bereits mehrere Gentherapie-Protokolle bekannt, die sich entweder noch im Stadium von Tierexperimenten (Alton et al. (1993) *(15)*; WO 93/1224 *(10);* Hyde et al. (1993) *(16)*, Debs et al. (1991) *(17)*) bzw. bereits in klinischen Studien am Patienten (Nabel (1993) *(18)*, (1994) *(19)*) befinden. In diesen Protokollen wird meist ein auf pBR322 bzw. pUC18/19 basierender Vektor verwendet, der als bakteriellen Selektionsmarker ein Ampicillinresistenzgen trägt.

Bei Applikation von Nukleinsäuren in einer gentherapeutischen Behandlung besteht die Möglichkeit, daß im Atmungs- und Verdauungstrakt und auf der Haut vorhandene Bakterien die Nukleinsäuren aufnehmen. Wenn der Marker jedoch ein aktives Antibiotikaresistenzgen (AB^{R}-Gen) ist, könnte dadurch als unerwünschter Nebeneffekt beim Patienten eine Antibiotikaresistenz erzeugt werden. Dies ist insbesondere von Nachteil bei der gentherapeutischen Behandlung der zystischen Fibrose. Hierbei werden dem Patienten große Mengen von Vektornukleinsäure als Plasmid-DNA, oder als Aerosol unter Verwendung von Liposomen als DNA-Transferreagenz appliziert (Alton et al. (1993) *(15)*).

Patienten, die an zystischer Fibrose erkrankt sind, leiden meist zusätzlich an bakteriellen Lungeninfektionen durch beispielsweise Pseudomonas aeruginosa, Staphylococcus aureus, Haemophilus influenzae, die üblicherweise durch Gabe von Antibiotika, wie Penicillin, behandelt werden. Eine Resistenz der Patienten gegen diese Antibiotika ist demnach nachteilig.

Die bislang beschriebenen Protokolle zur CF-Gentherapie mittels CFTR-Plasmid/Liposomen-Konjugaten bzw. Veröffentlichungen von in vitro oder Tierversuchen verwenden auf pUC18/19 bzw. pBR322 basierende Vektoren, welche als bakteriellen Selektionsmarker das Ampicillin-Resistenzgen enthalten (Alton et al. (1993) *(15)*; WO 93/1224 *(10)*; Hyde et al. (1993) *(16)*).

Auch in den anderen in vivo Gentherapie-Protokollen bzw. Veröffentlichungen von in vitro- oder Tiermodellstudien mit nackter DNA, bzw. DNA/Transfersystem-Konjugate werden die gängigen E. coli-Vektoren, basierend auf pUC- bzw. pBR-Basis mit dem Ampicillin-Resistenzgen (Nabel et al. (1993) *(18);* Lori et al. (1994) *(20);* Cotten et al. (1994) *(21)*; Lew et al. (1994) *(22)* etc.) verwendet.

Gegenstand der Erfindung ist die Verwendung einer zirkulären Vektor-DNA ohne Selektionsmarker - Gen zur Herstellung eines Arzneimittels zur Gentherapie von Säugetieren oder Menschen, wobei die Vektor - DNA dadurch erhältlich ist, dass ein Vektor ein Selektionsmarker-Gen und eine für den Vektor heterologe DNA-Sequenz enthält, die eine Modulation, Korrektur oder Aktivierung der Expression eines endogenen Gens oder die Expression eines durch die Vektor-DNA in die Zellen des Säugetieres oder des Menschen eingeführten Gens bewirkt,
a) unter Selektionsdruck amplifiziert und so gespalten wird, daß das genannte Selektionsmarker-Gen und die genannte heterologe DNA auf getrennten DNA-Fragmenten vorliegen,
b) das DNA-Fragment, welches die genannte heterologe DNA enthält, oder beide Fragmente zu Vektoren rezirkularisiert werden,
c) die genannten DNA-Fragmente vor oder nach der Rezirkularisierung getrennt werden,
d) das rezirkularisierte DNA-Fragment, welches die genannte heterologe DNA enthält, isoliert und
e) das so erhaltene rezirkularisierte DNA-Fragment zur Herstellung des Arzneimittels verwendet wird.

Die Spaltung in Schritt a) erfolgt vorzugsweise durch Restriktionsendonukleasen. In diesem Fall wird durch Zugabe von Ligase rezirkularisiert (Schritt b). Ebenso bevorzugt ist es, Spaltung und Rezirkularisierung in einem Schritt durch Rekombination mit Site-spezifischen Rekombinasesystemen (SSR) durchzuführen.

Die Verwendung von Site-spezifischen Rekombinase-Systemen (SSR-Systemen) ermöglicht es, bei richtiger Plazierung der spezifischen Rekombinations-Sites, das AB^{R}-Gen vom übrigen Vektoranteil (Plasmidreplikationsursprung und Insert) auf elegante Weise abzutrennen. Dazu müssen zwei spezifische Rekombinations-Sites stromaufwärts und stromabwärts des AB^{R}-Gens eingebaut werden. Wird eine SSR zugegeben, so führt diese eine spezifische Rekombination zwischen beiden Rekombinations-Sites durch, wodurch die DNA-Stücke zwischen den Rekombinations-Sites getrennt werden. Auf diese Weise entstehen zwei ringförmige Moleküle (eines mit dem AB^{R}-Gen und eines mit dem Insert und dem Plasmidreplikationsursprung), die beide je eine Rekombinations-Site tragen.

Es ist wesentlich, daß die DNA nach der Deletion des Vektoranteils wieder rezirkularisiert wird (durch Ligase oder Rekombinase), da zirkuläre DNA mit höherer Effizienz als lineare DNA transfizierbar ist (Chen and Okayama (1987) *(37)*) und eine höhere Halbwertszeit im Blut bzw. in der Zielzelle aufweist, d.h. weniger anfällig gegen Nukleaseeinwirkung ist.

Die so entstandenen zwei zirkulären Moleküle lassen sich durch chromatographische Methoden voneinander trennen. Je größer hierbei der Größenunterschied zwischen beiden Molekülen ist, desto besser funktioniert die Auftrennung. Die erhaltene zirkuläre therapeutische DNA enthält nur noch das therapeutisch wirksame Gen, plus notwendiger regulatorischer Elemente, um eine Genexpression in den humanen Zielzellen zu gewährleisten sowie aus technischen Gründen den E.coli-Plasmid-Replikationsursprung, der aber keinerlei störenden Einfluß hat. Das störende AB^{R}-Gen ist deletiert.

Die Site-spezifische Rekombination kann sowohl in vivo als auch in vitro durchgeführt werden. Bei der in vivo Site-spezifischen Rekombination wird ein in die Wirtszell-DNA (oder F-Episom) integriertes SSR-Gen induziert, das gebildete Genprodukt, die SSR, führt die spezifische Rekombinationsreaktion an dem zusätzlich in der Zelle vorhandenen therapeutischen Plasmid in vivo durch. Die Rekombinationsprodukte werden aus der Zelle isoliert und in chromatographischen Verfahrensschritten aufgetrennt. Zur Durchführung der Site-spezifischen Rekombination in vitro wird aufgereinigte SSR auf das über herkömmliche Methoden isolierte therapeutische Plasmid gegeben. Nach erfolgter Rekombination werden die zirkulären Endprodukte über chromatographische Verfahrensschritte voneinander getrennt.

Als SSR stehen prinzipiell drei Systeme zur Verfügung:
1. Die SSR-Systeme lysogener Phagen:
   z.B. das cre/lox-System des Bakteriophagen P1 (Sauer und Henderson (1988) *(44)*; Baubonis (1993) (46)
   das λint-System des Bakteriophagen λ (Landy et al. (1989) *(42)*)
   oder das Gin-System des Bakteriophagen Mu (Klippel et al. (1993) *(41)*).
2. Die SSR-Systeme des Hefeplasmids 2µ und analoger Plasmide aus anderen Hefestämmen:
   z.B. das "FLP/FRT"-System des 2µ-Episoms aus Saccharomyces cerevisiae (Cox et al. (1983) *(40)*),
   das "R" SSR-System des Episoms pSRI aus Zygosaccharomyces rouxi (Matsuzaki et al. (1990) *(43)*),
   das SSR-System des Episoms pKD1 aus Kluyveromyces drosophilarium (Chen et al. (1986) *(38)*)
   oder das SSR-System des Episoms pKW1 aus Kluyveromyces waltii (Chen et al. (1992) (39)).
3. Die Transposon-codierten Integrasen:
   z.B. die Integrase des Transposons Tn3 (Stark et al. (1992) *(45)*).

Besonders bevorzugt wird hierzu das cre/lox System des Bakteriophagen P1 verwendet (N. Sternberg et al. (1986) *(34)*; B. Sauer und N. Henderson (1989) *(35)*. Dazu enthält der Vektor an den 5'- und 3'-Enden der heterologen DNA lox^{P} Sites. Die Rekombination (entsprechende Spaltung und Rezirkularisation) erfolgt durch das cre Genprodukt Rekombinase. Es entstehen zwei zirkuläre Plasmidfragmente (mit und ohne heterologe DNA), die bei ausreichenden Größenunterschieden z.B. chromatographisch getrennt werden können. Vorzugsweise ist der Vektor so zusammengesetzt, daß der Anteil der heterologen DNA und der Anteil des Basisvektors sich in ihrer Größe um mehr als den Faktor 1,5, vorzugsweise 2, unterscheiden. Die Rezirkularisierung ist wesentlich, da zirkuläre DNA mit höherer Effizienz transformierbar ist und eine höhere Halbwertszeit im Blut bzw. der Zielzelle aufweist als lineare DNA (geringe Nukleasenempfindlichkeit).

Vorzugsweise wird das Arzneimittel als Aerosol angewendet.

Besonders bevorzugt wird eine Vektor-DNA verwendet, durch die ein defektes Gen korrigiert, ein intaktes Gen eingeführt oder am korrekten Genlocus ausgetauscht werden kann. Unter einer Vektor-DNA im Sinne der Erfindung ist ein nichtvirales DNA Molekül, auf Basis eines prokaryontischen Plasmids, zu verstehen. Zusätzlich enthält dieses DNA-Molekül die in gentherapeutische Verfahren zu übertragende DNA, vorzugsweise ein exprimierbares Gen.

Als nichtvirale DNA im Sinne der Erfindung ist zu verstehen, daß diese DNA nicht Bestandteil eines infektiösen viralen Partikels ist und kein intaktes virales Genom enthält. Die nichtvirale DNA kann jedoch virale Sequenzen, wie z. B. Regulationssequenzen (z. B. Promotor, Enhancer), Transkriptionsstopps oder virale Gene, wie z. B. das Herpes Simplex Tk-Gen, enthalten.

Besonders bevorzugt ist die Anwendung derartiger Vektor-DNA zur Behandlung der zystischen Fibrose am Menschen. Ein hierfür geeignetes Gen ist beispielsweise in der WO 91/02796 *(11)* beschrieben. Dort ist auch die Herstellung und Anwendung von Vektoren zur gentherapeutischen Behandlung der zystischen Fibrose beschrieben.

Die DNA-Vektoren sind insbesondere zu solchen gentherapeutischen Behandlungen geeignet, bei denen die Vektoren unmittelbar mit Oberflächen in Säugetieren oder Menschen in Berührung kommen. Derartige Oberflächen sind beispielsweise der Atmungs- und der Verdauungstrakt sowie die Hautoberfläche.

Ein weiterer Gegenstand der Erfindung ist eine zirkuläre Vektor-DNA-Präparation in einer Menge von 300 bis 500 µg Plasmid (30 bis 90 pmol), welche ein Gen oder Genfragment enthält, das die Aktivierung, Modulation oder Korrektur der Expression eines endogenen zystischen Fibrosegens (CFTR-Gen, cystic fibrosis transmembrane conductance regulator gene) in Säugerzellen bewirkt oder ein CFTR-Gen enthält, welches nach Transfektion von Säugerzellen mit der Vektor-DNA die Expression dieses Gens bewirkt, dadurch gekennzeichnet, daß dieser Vektor
a) unter Selektionsdruck amplifiziert, so gespalten wird, daß das genannte Selektionsmarker-Gen und die genannte heterologe DNA auf getrennten DNA-Fragmenten vorliegen,
b) die genannten DNA-Fragmente vor oder nach der Rezirkularisierung getrennt werden,
c) das DNA-Fragment, welches die genannte heterologe DNA enthält, oder beide Fragmente zu einem Vektor rezirkularisiert,
d) das rezirkularisierte DNA-Fragment, welches die genannte heterologe DNA enthält, isoliert und
e) das so erhaltene rezirkularisierte DNA-Fragment zur Herstellung des Arzneimittels verwendet wird.

Nach Isolierung kann die Vektor-DNA-Präparation lyophilisiert oder in einer Pufferlösung (z.B. TE-Puffer) gelagert werden.

Erfindungsgemäß geeignete Nukleinsäuren können nach Verfahren hergestellt werden wie z.B. in Sambrook et al. (1985) *(47)* beschrieben. Es können aber auch Anionen-Austauschsäulen zur Trennung der DNA von RNA und Proteinen verwendet werden (z.B. Qiagen Plasmidreinigungs-Kit).

Wesentlicher Anwendungszweck ist die verbesserte gentherapeutische Behandlung von zystischer Fibrose. Die bisher bekannten Verfahren zur Behandlung von zystischer Fibrose sind beispielsweise in der WO 91/2796 *(11)* beschrieben. Dort ist auch ein für die Gentherapie geeignetes CFTR-Gen beschrieben.

Die zystische Fibrose ist eine schwerwiegende monogenetische, autosomal rezessive Erbkrankheit, die mit einer Frequenz von 1/2500 Geburten auftritt. Sie ist charakterisiert durch einen mangelhaften Elektrolyttransport der Epithelgewebsmembranen, was zu Funktionsabnormalitäten (Dysfunktion exokriner Drüsen) im Respirationstrakt, Pankreas (vermehrte Produktion und erhöhte Viskosität des Sekretes mucöser Drusen), Schweißdrüsen (erhöhter Elektrolytgehalt des Schweißes und damit verbundener Flüssigkeits- und Elektrolytverlust) und Gonaden führt. Die respiratorische Insuffizienz aufgrund einer unzureichenden Sekretion von Chloridionen in den Bronchialschleim durch Zellen des Atmungsorgan-Epithels, stellt die häufigste klinische Manifestation und Todesursache bei CF-Patienten dar. Das verantwortliche Gen konnte kloniert und das Genprodukt charakterisiert werden als "cyclic adenosine monophosphate" (cAMP) abhängiges Chloridionenkanalprotein (CFTR = Cystic Fibrosis Transmembrane Conductance Regulator) (WO 91/02796 *(11)*). Das Wissen über die Pathophysiologie der Krankheit, die Struktur und Funktion von CFTR und Mutationen im Zusammenhang mit den CFTR-Funktionsstörungen machen es heute möglich, neben den klassischen, nicht sehr wirkungsvollen Therapieansätzen, verschiedene Gentherapieansätze durchzuführen.

In angemeldeten bzw. laufenden klinischen Protokollen zur CF-Therapie werden bisher zwei Wege beschritten. Nach dem ersten Verfahren wird das CFTR-Gen mittels Inhalation von CFTR-Adenovirusvektoren angewendet. Adenoviren infizieren natürlicherweise das Lungenepithel. Mit dieser Methode wurden schon erste klinische Erfolge erzielt, aber nur für eine kurze Zeitspanne von wenigen Wochen und mit ungewünschten toxischen Nebenwirkungen (Zabner & Welsh (1993) *(23)*. Die zweite Methode beinhaltet die Einbringung von CFTR-Plasmiden komplexiert mit kationischen Liposomen mittels Inhalation in den Respirationstrakt (Alton et al. (1993) *(15)*). Hierbei werden in Mäusen ca. 1 mg Plasmid-DNA / Maus verabreicht; bei Menschen werden die Plasmidgaben im Bereich von 100 µg - 1 mg, vorzugsweise 300 - 500 µg Plasmid / Patient liegen, was bei einer Plasmidgröße von 8,2 kb (Alton et al. (1993) *(15)*; Whitsett et al. (1992) *(24)*) einer Zahl von ca. 5 x 10¹³ DNA-Molekülen entspricht. Diese Applikation und Dosierung ist auch erfindungsgemäß bevorzugt.

Die Lungenepithelzellen können nur einen kleinen Anteil der eingebrachten Plasmidmenge aufnehmen. Es ist zu erwarten, daß der größte Anteil des Plasmids entweder ausgeatmet oder vom Patienten verschluckt wird, daß also ein hoher Anteil in die Umgebung (Patienten in Unterdrucksicherheitsräumen) und den Magen/Darm-Trakt des Patienten gelangt.

In der Lungenflora befinden sich verschiedene Bakteriengattungen, von denen einige als opportunistische Pathogene in Erscheinung treten können, z.B. Pseudomonaden, Haemophilus, Enterobacteriaceae, Staphylokokken etc. (Balows (1991) *(12)*.

Eine bakterielle Besiedelung des, bei CF-Patienten im Bereich der Atemwege verstärkt sezernierten, viskosen, eiweißreichen Sekrets durch Bakterien ist eine häufige Ursache schwerer Fälle von Bronchitis und Lungenentzündung. CF-Patienten sind vor allem Infektionen der Bronchien und Lungen durch Haemophilus influenzae, Pseudomonas aeruginosa und Staphylococcus aureus ausgesetzt (Dodge et al. (1993) (*25*), FitzSimmons (1993) (*26*)), weshalb sie in häufiger Folge Antibiotikabehandlungen unterzogen werden müssen.

Wichtigste Antibiotika hierfür sind Penicillin bzw. dessen Derivate, wie z. B. Ampicillin (H. influenzae) und Carbenicillin (P. aeruginosa, β-Lactamase sensitives Penicillinderivat, Davis et al. (1980) (*27*)). Wegen weitverbreiteter Penicillinresistenzen bei Staphylococcus aureus sind hier vor allem die β-Lactamase-resistenten Penicillinderivate (Methicillin, Oxacillin, Cephalosporin) von Bedeutung.

Daneben sind bei Lungeninfektionen noch andere Erreger von Bedeutung, z.B. Streptococcus pneumoniae (= Pneumokokken), der häufigste Erreger von bakterieller Lungenentzündung und Enterobacteriaceae (z.B. Klebsiella pneumoniae), wobei besonders bei Pneumokokken Penicillin das wichtigste Therapeutikum ist (Davis et al (1980) *(27)*).

Im Magen/Darm-Trakt sind unter anderem Enterobacteriaceae und Enterokokken vorhanden (Balows et al. (1991) *(12)*). Bei der Behandlung von Darminfektionen, welche durch Enterobacter, E. coli, Serratia und Streptococcus faecalis verursacht sind, spielen Penicilline und deren Derivate ebenfalls eine zentrale Rolle (Davis et al. (1980) *(27)*).

Bereits 1944 wurde von Avery die Aufnahme von hochmolekularer DNA durch Pneumokokken aus dem Medium beschrieben, ein Vorgang, der als natürliche Kompetenz bezeichnet wird und in der bakteriellen Evolution eine wichtige Rolle spielt, daher im Bakterienreich weit verbreitet ist. Physiologische Transformation wurde bei den Gattungen Haemophilus, Streptococcus, Staphylococcus, Neisseria, Bacillus and Acinetobacter (Davis et al. (1980) *(27)*) beobachtet. Es ist anzunehmen, daß auch Pseudomonaden, bei denen der horizontale Gentransfer weit verbreitet ist, hochmolekulare DNA aus dem Medium aufnehmen können.

Bakterien der natürlichen Flora des Menschen (Respirationstrakt, Magen/Darmtrakt, Haut, Schleimhäute, Auge etc.) sind damit in der Lage, Plasmid-DNA aufzunehmen. Durch rekombinatorische Ereignisse kann die aufgenommene DNA in die zelleigene DNA (Chromosom, Plasmide) integrieren und dabei unter die Kontrolle eines wirtseigenen Promotors geraten, d.h. exprimiert werden.

Bei der Verabreichung von ca. 300 - 500 µg Plasmid-DNA / CF-Patient (entspricht bei einer Plasmidgröße von 8,2 kb ca. 5 x 10¹³ Molekülen; Alton et al. (1993) *(15)* besteht die Gefahr, daß unter den Bakterien der Lungenflora, aber auch der anderen Körperregionen Antibiotikaresistente Keime entstehen. Wie bereits ausgeführt, ist gerade bei CF-Patienten, die besonders unter bakteriellen Lungeninfektionen leiden und ständig mit Antibiotika cotherapiert werden müssen, eine Erzeugung von Antibiotika-Resistenzen, im besonderen Maße einer Ampicillin-Resistenz (β-Lactamase) verhängnisvoll, besonders, da die bisher verwendeten Gentherapieverfahren noch keine vollständige Heilung bzw. keine persistierende Korrektur bewirken.

Es ist außerdem nicht auszuschließen, daß das mit dem CFTR-Plasmid eingebrachte Ampicillin-Resistenzgen in die Patienten-DNA integriert, dort unter der Kontrolle eines zelleigenen Promotors exprimiert und das Genprodukt aktiv oder auch passiv (z.B. Zellyse bei Entzündungsreaktionen) ausgeschleust wird. Die lokal freigesetzte β-Lactamase könnte auch im Falle einer allgemeinen bakteriellen Infektion eine Penizillin-Therapie behindern.

Vorteilhaft ist die erfindungsgemäße Verwendung von DNA-Vektoren ohne Selektionsmarker-Gen bei der gentherapeutischen Behandlungen von AIDS- (Lori et al. (1994) *(20)*) bzw. Krebs-Patienten (Nabel et al. (1993) *(18)*), da in beiden Fällen die Patienten in der Regel, durch das Krankheitsbild an sich (bei AIDS) bzw. durch Chemotherapeutika-Therapien oder Radiotherapien (bei Krebs) immunsupprimiert sind. Bei diesen Patienten können bakterielle Infektionen durch Antibiotika-Behandlung verhindert bzw. unter Kontrolle gebracht werden.

Neben der Lunge bzw. dem Respirationstrakt müssen auch gentherapeutische Ansätze zur Behandlung anderer Gewebe unter den oben erwähnten Gesichtspunkten betrachtet werden:

Muskelgewebe: Gentherapeutische Plasmide können z.B. zur in vivo Vaccinierung direkt ins Muskelgewebe (Ulmer et al. (1993) *(28)*; Davis et al. (1993) *(29)*); Lew et al. (1994) *(22)*) oder in Tumore (Immunstimulation zur Tumorvaccinierung; Nabel et al. (1993) *(18)*; San et al. (1993) *(30)* injiziert werden. Therapeutische Plasmide wurden dazu bislang in geringen Dosen injiziert, weshalb die injizierte Plasmid-DNA nur um den Stichkanal lokalisiert blieb. Um systemische Reaktionen zu erhalten, ist eine höhere Dosierung bzw. eine systemische Verabreichung der therapeutischen Plasmide nicht zu umgehen. Dies hat dann eine Verbreitung der Plasmid-DNA im Blutsystem zur Folge.

Blutsystem: Zur Gentherapie in der Leber können Konjugate von Plasmid-DNA/Polylysin/Lebertargeting-Gruppen (Chiou et al. (1994) *(31)*) intravenös verabreicht werden. Dies hat eine Ausbreitung der Plasmid-DNA im Blutsystem zur Folge.

In beiden Fällen (Muskelgewebe, Blutsystem) könnten die eingebrachten Plasmide an Bakterienherde gelangen, von diesen aufgenommen werden und die Antibiotika-Therapie im Falle des Ausbruchs einer Infektion behindern.

Dann: Zur Gentherapie von Colon-Cancer können Konjugate aus therapeutischem Plasmid (z.B. mit Tumor Suppressor-Gen) direkt in den Darm eingebracht werden (Arenas et al. (1994) *(32)*). Ebenso wird über Gene-Delivery z.B. von Genen, welche für den LDL-Rezeptor kodieren, über die Darmschleimhaut nachgedacht. Eingebrachte Plasmide können das Antibiotika-Resistenzgen auf die Bakterien des Magen/Darm-Traktes übertragen.

Haut: Plasmid-DNA kann direkt als Konjugat mit Liposomen in Hautzellen, z.B. zur Gentherapie von Melanom oder Hämophilie B (Faktor IX, X) aufgenommen werden (Alexander et al. (1994) *(33)*). Auf diese Weise können Bakterien der Hautflora Antibiotika-Resistenzen erhalten.

Auge: Persistierende Virusinfektionen des Auge können durch Gentherapie mittels therapeutischen Plasmiden therapiert werden, was die Gefahr der Übertragung von Antibiotika-Resistenzen auf die Bakterien der Augenflora birgt.

Die nachfolgenden Beispiele, die Abbildung und das Sequenzprotokoll erläutern die Erfindung weiter. Eine ausführliche Beschreibung der experimentellen Bedingungen ist in J. Sambrook (1989) *(13)* enthalten.

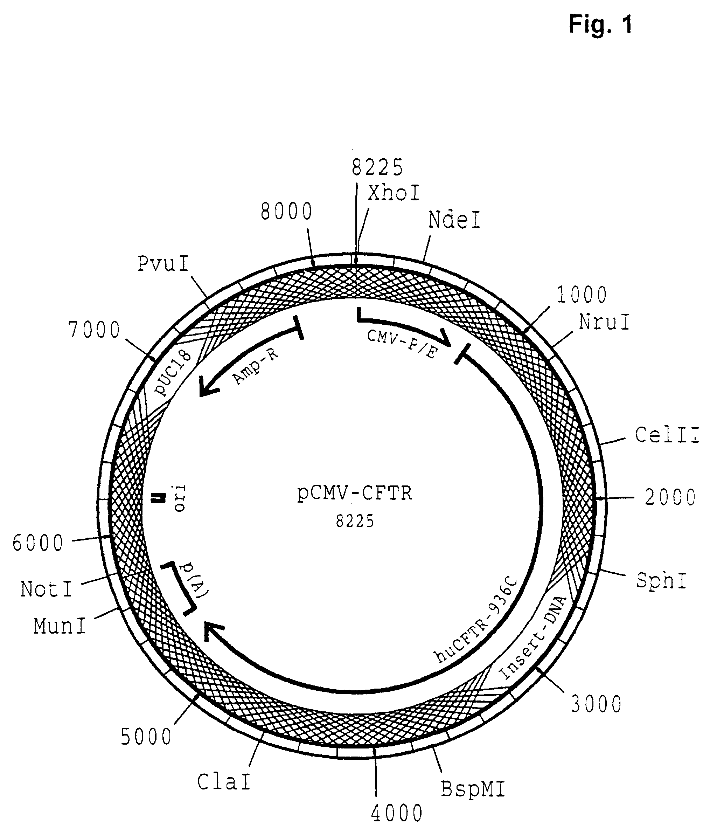

- **Figur 1**: zeigt die Plasmidkarte von pCMV-CFTR.
- **SEQ ID NO:1**: zeigt die DNA-Sequenz von pCMV-CFTR.
- **SEQ ID NO:2-5**: zeigen DNA-Sequenzen von DNA-Linkern.

### Beispiel 1

### Bestimmung der Aufnahmerate von Plasmiden und Erzeugung von Antibiotikaresistenzen durch Bakterien der humanen Lungen- und Darmflora

Es soll gezeigt werden, daß E. coli Plasmid-DNA z.B. pUC18 bzw. Derivate davon von Bakterien anderer Gattungen aufgenommen und das Ampicillinresistenzgen exprimiert werden kann, was zu ampicillinresistenten Klonen führt. Die Wahrscheinlichkeit der Entstehung ampicillinresistenter Keime wird bestimmt durch die Aufnahmerate der Plasmid-DNA aus dem Substrat und der Einbaurate des Ampicillinresistenzgens durch nichthomologe Rekombination in die chromosomale DNA des Wirtsbakteriums. Plasmide mit alternativen Markern bzw. durch Stop-Codons inaktivierten Markern (siehe oben) rufen keine Resistenzbildung mehr hervor.

Die Untersuchungen werden an folgenden Bakterienarten durchgeführt:
Gram-negative:
   Haemophilus influenzae
   Pseudomonas aeruginosa
   Klebsiella pneumoniae
   Escherichia coli (WT)
Gram-positive:
   Staphylococcus aureus
   Streptococcus pneumoniae

Dazu werden verschiedene Mengen Plasmid-DNA unter verschiedenen Bedingungen zu Kulturen der oben genannten Organismen zugegeben.

Es werden Flüssigkulturen in Nährmedium, unter Verwendung von isotonischem Puffer (NaCl, Mg²⁺, Ca²⁺) durchgeführt. Diesen wird Plasmid-DNA (z.B. pUC18/ap^{R}-Gen) in verschiedenen Konzentrationen zugegeben. Nach Inkubation über mehrere Stunden unter Schütteln, wird Antibiotikum zugegeben. Es wird weiter inkubiert, ausplattiert auf einem antibiotikahaltigen Agar und eine Verdünnungsreihe auf nicht antibiotikahaltigem Agar durchgeführt. Die Lebendkeimzahl wird bestimmt nach Inkubation durch Auszählen und Identifizieren bzw. Charakterisieren der erhaltenen Kolonien.

Hierzu wird im einzelnen eine über Nacht inkubierte Bakterienkultur abzentrifugiert, das Pellet mit Phosphat-gepufferter Salinelösung (PBS) gewaschen, in 2% des Kulturvolumens PBS resuspendiert und mit 200 ng Plasmid-DNA/ml versetzt. Es wird sofort eine Verdünnungsreihe auf Platten ohne Antibiotikum ausplattiert. Die Suspension wird bei 37°C langsam geschüttelt und zu verschiedenen Zeiten (1, 2 und 4 h) werden Verdünnungsreihen auf Standard-I-Agar mit und ohne Antibiotikum (Ampicillin 100 µg/ml) ausplattiert und bei 37°C bebrütet. Auf Antibiotikaplatten werden die Verdünnungen 10¹-10⁵, auf Platten ohne Selektion werden die Verdünnungen 10⁹-10¹⁰ ausplattiert. Am nächsten Tag werden die transformierten resistenten Kulturen ausgezählt und im Verhältnis zu der Lebendkeimzahl gesetzt.

Alternativ wird eine definierte Plasmidmenge und Bakterien definierter Keimzahl auf Nähragar ausplattiert und einige Stunden inkubiert, Antibiotika durch Aufsprühen zugegeben und die erhaltenen Kolonien ausgezählt und identifiziert bzw. charakterisiert. Ebenso kann eine definierte Plasmidmenge und Bakterien definierter Keimzahl auf einem Nylonfilter aufgetragen werden, die Filter einige Stunden auf Nähragar inkubiert werden. Anschließend werden die Filter auf antibiotikahaltigem Nähragar umgesetzt, inkubiert und die erhaltenen Kolonien ausgezählt und identifiziert bzw. charakterisiert.

### Beispiel 2

### Herstellung und Amplifikation von Sicherheitsvektoren auf Basis von pUC18, die keinen Selektionsmarker mehr besitzen

Das Prinzip des Verfahrens beruht auf Restriktion des isolierten, therapeutischen Plasmids, Abtrennung der kleinen Fragmente mit dem Vektoranteil (+ Resistenzgen) und Religation des großen Fragmentes mit dem therapeutisch wirksamen Gen in mehreren Schritten in vitro (Restriktionsendonukleasen + Ligase) oder in einem Schritt in vivo oder in vitro (Site-spezifische Rekombinasen). Die Durchführbarkeit des Verfahrens wurde anhand des Vektors pUC 18 und des therapeutischen Plasmids pCMV-CFTR (Alton et al. *(15)*) gezeigt.

Die Plasmidkarte von pCMV-CFTR ist in Figur 1, die DNA-Sequenz in SEQ ID NO:1 gezeigt.

### Plasmidkonstruktion

**1.** Um die Abtrennbarkeit des Inserts (mit dem therapeutischen Gen) vom Vektoranteil zu erleichtern, wurden, über den Vektor verteilt, mehrere Linker mit Restriktionsschnittstellen eingebaut. Als Restriktionsenzyme wurden PstI und PvuI gewählt, da sie im Beispiel bezogenen Plasmid pCMV-CFTR nicht im Insert (CMV-CFTR) schneiden. Es können aber auch andere Enzyme, die die gleiche Eigenschaft haben, nicht im Insert zu schneiden, verwendet werden. Ebenso können auch Linker mit mehr als nur einer Restriktionsschnittstelle verwendet werden. Im Beispiel wurden Linker mit 2 Restriktionsschnittstellen, und zwar für PstI und PvuI, verwendet.
   So wurden in pUC 18 mittels laborüblicher Techniken in die singulären AflII-, AlwNI- und AatII-Schnittstellen synthetische DNA-Linker mit den Erkennungssequenzen von PvuI + PstI in definierter Reihenfolge eingebaut (5'PstI-PvuI in AatII und AflIII; 5'Pvu-PstI in AlwNI).
   Die Reihenfolge ist vor allem bei der AatII und der AlwNI-Schnittstelle wichtig, da damit eine zusätzliche Sicherheit eingebaut ist, die neben der physikalischen Abtrennung verhindert, daß das Amp^{R}-Gen mit dem Insert religiert. Daher müssen die Doppelstrang-Linker für jede jener Enzymschnittstellen entsprechende Überhänge haben, um sie gerichtet zu klonieren, bzw. bei der Klonierung der Linker über stumpfe Enden muß nach Klonen mit der richtigen Orientierung gesucht werden.
**2.** Der Polylinker von pUC18 wurde gegen einen neusynthetisierten Polylinker ausgetauscht, um erstens die Klonierung der CMV-CFTR-Genkassette zu erleichtern und zweitens eine saubere Abtrennung von Vektor und Insert (= CMV-CFTR-Genkassette) zu ermöglichen.
   Der synthetische DNA-Polylinker enthält die Restriktionsschnittstellen in folgender Reihenfolge:
   5'-(HindIII)-PvuI-PstI-XhoI-SaII-XbaI-BamHI-SmaI-NotI-PstI-PvuI-(EcoRI).
   Die Klammer bedeutet, daß der Polylinker Überhänge für die entsprechenden Enzyme hat und so gerichtet in pUC18, der mit HindIII und EcoRI geschnitten wurde, gerichtet ligiert werden kann.
   Der entstandene Vektor erhielt die Bezeichnung pUC-PP_1.
**3.** Zwischen die XhoI- und die NotI-Schnittstellen von pUC-PP_1 wurde das 5,8 kb große DNA-Fragment mit der CMV-CFTR-Genkassette, das ebenfalls über einen XhoI/NotI-Doppelverdau aus pCMV-CFTR herausgeschnitten wurde, gerichtet eingebaut (Zugabe von Ligase). Das entstandene Plasmid erhielt die Bezeichnung pCMV-CFTR_PP2. Es zerfällt bei einer Restriktion mit PvuI + PstI in Fragmente der Größen: 5,8 und 0,85, 0,55, 0,42, 0,36, 0,34, 0,12 kb.

### Herstellung des resistenzfreien Plasmids

### a) Spaltung mit Restriktionsendonukleasen

pCMV-CFTR_PP2 wurde in E.coli transformiert. E.coli (pCMV-CFTR_PP2) wurde fermentiert, aufgeschlossen und die Plasmid DNA über eine QIAGEN®-Säule nach Spezifikation des Herstellers (QUIAGEN, Deutschland) gereinigt. Die gereinigte Plasmid DNA wurde mit PvuI geschnitten (10 U/µg; 1 µg in 20 µl Volumen; nach Spezifikation des PvuI-Herstellers). Die DNA wurde dann mit PvuI + PstI nachgeschnitten (5 U/µg PvuI + 10 U/µg PstI). Die Fragmente wurden über eine Molekularsiebsäule aufgetrennt und das viel größere 5,8 kb-Fragment dadurch abgetrennt. Das eluierte 5,8 kb-Fragment wurde mittels T4-Ligase (nach Spezifikation des Herstellers) religiert. Die 5,8 kb große rezirkularisierte DNA (1 mg DNA in 1 ml Puffer) mit der CMV-CFTR Genkassette wurde nochmals über eine QIAGEN-Säule gereinigt und in wenig Puffer Tris HCl/EDTA Puffer (10 mmol/l Tris HCI, 0.1 mmol/l EDTA (pH = 7,0)) aufgenommen.

### b) Spaltung und Rezirkularisierung über Rekombination

### ba) in vivo Anwendung des cre/lox Systems zur rekombinanten Deletion des AB^{R}-Gens:

Die Arbeiten wurden analog zu den von Sauer (1988) *(44)* beschriebenen Methoden über die cre-Rekombinase vermittelte Freisetzung zirkulärer Plasmide, welche von loxP-Sites flankiert in großen linearen DNa-Molekülen integriert vorlagen, durchgeführt. In diesen Arbeiten wurden allerdings E.coli Stämme verwendet, in denen das cre-Rekombinase-Gen konstitutiv exprimiert vorlag. Für das hier beschriebene Beispiel muß das cre-Rekombinase-Gen unter der Kontrolle eines strikt regulierbaren Promotors vorliegen.

Dazu wurden dem therapeutischen Plasmid pCMV-CFTR stromaufwärts und stromabwärts des Ampicillin-Resistenzgens zwei 34 bp lange loxP-Rekombinations-Sites eingebaut. Das Plasmid erhielt die Bezeichnung pCMV-CFTR_2loxP. Das erhaltene Plasmid pCMV-CFTR_2loxP wurde in E.coli XL1-Blue_λimm434nin5_X1-T5-cre (Konstruktion analog Sauer (1988) *(44)*) kloniert, der den lysogenen Phagen λ mit dem cre-Rekombinase-Gen unter der Kontrolle des strikt regulierbaren T5-Promotors (Bujard et al. (1987) *(36)*) sowie ein laclq-Gen (F-episomal codiert) enthält.

### bb) Anzucht und in vivo Rekombination

E.coli Xl1-blue_λimm434nin5_X1-T5-cre (pCMV-CFTR_2loxP) wurde in LB-Medium mit 100 µg/ml Ampicillin angezogen. Der T5-Promotor wurde durch Zugabe von 5 mM IPTG induziert und damit die Expression des cre-Rekombinase-Gens angeschaltet. Die cre-Rekombinase führte in vivo eine Rekombination der beiden loxP-Sites in pCMV-CFTR_2loxP durch. Dadurch wurde das Plasmid pCMV-CFTR_loxP in zwei zirkuläre Plasmide stark unterschiedlicher Größe umgewandelt. Anschließend wurden die E.coli-Zellen geerntet, aufgeschlossen und das Gemisch an zirkulären Plasmiden isoliert. Das Gemisch bestand aus unverändertem pCMV-CFTR_2loxP, dem zirkulären Amp^{R}-Gen mit einer loxP-Site und dem gewünschten Endprodukt (der zirkulären DNA mit dem therapeutischen Insert + einer loxP-Site + dem pUC-Replikationsursprung). Anschließend wurden die zirkulären DNA-Moleküle unterschiedlicher Größe mittels chromatographischer Methoden aufgetrennt und das gewünschte Endprodukt in Citrat-Puffer (pH = 7,0) aufgenommen.

Da die cre/lox-Rekombination in beide Richtungen laufen kann, ist es wichtig, die Reaktionszeit bzw. den Erntezeitpunkt nach IPTG-Induktion zu optimieren. Da das T5-Promotorsystem selbst in lacl^{q}-Stämmen nicht vollständig reprimiert vorliegt, kommt es immer zu einer niedrigen Expression des cre-Rekombinase-Gens und damit zu einem niedrigen Level an Rekombinationen. Durch Plazierung einer der zwei loxP-Sites zwischen das Amp^{R}-Gen und den Replikationsursprung ist jedoch sichergestellt, daß durch die cre-vermittelten Rekombinationen nur Konstrukte entstehen, welche nicht replizieren können bzw. keine Ampicillin-Resistenz mehr vermitteln. Sie gehen während den Replikationszyklen unter einem Selektionsdruck durch Ampicillin-Zugabe schnell verloren.

### Referenzliste

(1) T. Friedmann, Science 244 (1989) 1275
(2) Morgan 1993, RAC DATA MANAGEMENT Report, June 1993
(3) Mulligan, R.C. (1991) in Nobel Symposium 8: Ethiology of human disease at the DNA level (Lindsten, J. and Pattersun Editors), pages 143 - 189 Raven Press
(4) McLughlin, J. Virol. 62 (1988) 1963
(5) Moss et al., Ann. Rev. Immunol. 5 (1987) 305
(6) Rasmussen et al., Methods Enzymol. 139 (1987) 642
(7) Margolskee et al., Mol. Cell. Biol. 8 (1988) 2937
(8) Felgner et al., Proc. Natl. Acad. Sci. USA 84 (1987) 7413
(9) Davies et al., Ann. Rev. Microbiol. 32 (1978) 469
(10) WO 93/1224
(11) WO 91/02796
(12) Balows, Manual of Clinical Microbiology (1991)
(13) J. Sambrook, Molecular Cloning, Second Edition (1989) Cold Spring Harbor Laboratory Press, New York
(14) Wagner et al., Proc. Natl. Acad. Sci. USA 87 (1990) 3410-3414
(15) Alton et al., Nature Genetics 5 (1993) 135-142
(16) Hyde et al., Nature 362 (1993) 250-255
(17) Debs et al., WO 92/1224, A1, 17.12.91 US
(18) Nabel et al., Proc. Natl. Acad. Sci. USA (1993) 11307-11311
(19) Nabel et al., Human Gene Therapy 5 (1994) 79-92
(20) Lori et al., Gene Therapy 1 (1994) 27-31
(21) Cotten et al., J. of Cellular Biochemistry 18A (1994) Abstract DZ002
(22) Lew et al., J. of Cellular Biochemistry 18A (1994) Abstract DZ120
(23) Zabner & Welsh, Cell 75 (1993) 207-216
(24) Whisett et al., Nature Genetics 2 (1992) 13-20
(25) Dodge et al., Cystic Fibrosis, Current Topics, Vol. 1(1993), John Wiley & Sons
(26) FitzSimmons, S.C., The Journal ofPediatrics 122 (1993) 1-9
(27) Davis et al., Microbiology, Third Edition (1980) Harper International Edition
(28) Ulmer et al., Science 259 (1993) 1745-1749
(29) Davies et al., Human Gene Therapy 4 (1993) 733-740
(30) San et al., Human Gene Therapy 4 (1993) 781-788
(31) Chiou et al., J. of Cellular Biochemistry 18A (1994) Abstract DZ109
(32) Arenas et al., J. of Cellular Biochemistry 18A (1994) Abstract DZ101
(33) Alexander et al., J. of Cellular Biochemistry 18A (1994) Abstract DZ400
(34) Sternberg, N., et al., J. Mol. Biol. 187 (1986) 197-212
(35) Sauer, B., Henderson, N., NAR 17 (1989) 147-161
(36) Bujard, H., et al., Methods in Enzymology 155 (1987) 416-443
(37) Chen, C. und Okayama, Mol. and Cell. Biol. 7 (1987) 2745-2752
(38) Chen, X.J., et al., NAR 14 (1986) 4471-4481
(39) Chen, X.J., et al., J. of Gen. Microbiol. 138 (1992) 337-345
(40) Cox, M.M., Proc. Natl. Acad. Sci. USA 89 (1983) 4223-4227
(41) Klippel, A., et al., The EMBO Journal 12 (1993) 1047-1057
(42) Landy, A., Ann. Rev. Biochem. 58 (1989) 913-949
(43) Matsuzaki, H., et al., J. of Bacteriol. 172 (1990) 610-618
(44) Sauer, B., and Henderson, N., Gene 70 (1988) 331-341
(45) Stark, W.M., et al., TIG 8 (1992) 432-439
(46) Baubonis et al., NAR 21 (1993) 2025-2029
(47) Sambrook et al., CSH Press (1985), Cold Spring Harbor, 1.21-1.52

## Patentansprüche

1. Verwendung einer zirkulären Vektor-DNA ohne Selektionsmarker - Gen zur Herstellung eines Arzneimittels zur Gentherapie von Säugetieren oder Menschen, wobei die Vektor - DNA dadurch erhältlich ist, dass eine Vektor-DNA, welche ein Selektionsmarker-Gen und eine für den Vektor heterologe DNA-Sequenz enthält, die eine Modulation, Korrektur oder Aktivierung der Expression eines endogenen Gens oder die Expression eines durch die Vektor-DNA in die Zellen des Säugetiers oder des Menschen eingeführten Gens bewirkt,
a) unter Selektionsdruck amplifiziert und so gespalten wird, daß das genannte Selektionsmarker-Gen und die genannte heterologe DNA auf getrennten DNA-Fragmenten vorliegen,
b) das DNA-Fragment, welches die genannte heterologe DNA enthält, oder beide Fragmente zu Vektoren rezirkularisiert werden,
c) die genannten DNA-Fragmente vor oder nach der Rezirkularisierung getrennt werden,
d) das rezirkularisierte DNA-Fragment, welches die genannte heterologe DNA enthält, isoliert und
e) das so erhaltene rezirkularisierte DNA-Fragment zur Herstellung des Arzneimittels verwendet wird.

2. Verwendung nach Anspruch 1, **dadurch gekennzeichnet, daß** die Spaltung in Schritt a) durch eine Restriktionsendonuklease erfolgt.

3. Verwendung nach Anspruch 1, **dadurch gekennzeichnet, daß** die Spaltung in Schritt a) und die Rezirkularisierung in einem Schritt durch eine sitespezifische Rekombination erfolgt.

4. Verwendung nach Anspruch 1 oder 3, **dadurch gekennzeichnet, daß** sich an die 3'- und 5'-Enden der heterologen DNA jeweils eine LoxP-Site anschließt und die Schritte a) und b) durch die Rekombinase Cre bewirkt werden.

5. Verwendung nach den Ansprüchen 1 bis 3, **dadurch gekennzeichnet, daß** die gentherapeutische Behandlung in den Atmungswegen, in dem Verdauungstrakt oder auf der Hautoberfläche erfolgt.

6. Verwendung nach Anspruch 5, **dadurch gekennzeichnet, daß** die zirkuläre Vektor-DNA eine Modulation oder Aktivierung der Expression des endogenen Gens für die zystische Fibrose bewirkt oder für das zystische Fibrose-Gen codiert.

7. Verwendung nach Anspruch 6, **dadurch gekennzeichnet, daß** das Arzneimittel als Aerosol zur Anwendung im Atmungstrakt hergestellt wird.

8. Therapeutische Präparation einer zirkulären Vektor-DNA in einer Menge von 30 bis 90 pmol, die eine für den Vektor heterologe DNA-Sequenz enthält, die die Modulation, Aktivierung oder Korrektur eines endogenen zystischen Fibrose-Gens in Säugerzellen bewirkt, oder welche ein in Säugerzellen exprimierbares zystisches Fibrose-Gen enthält, **dadurch gekennzeichnet, daß** diese Vektor-DNA kein Selektionsmarker-Gen enthält.

## Claims

1. Use of a circular vector DNA without a selection marker gene to produce a drug to treat mammals or humans by gene therapy, wherein the vector DNA can be obtained by a method, **characterized in that** a vector DNA which contains a selection marker gene and a DNA sequence that is heterologous for the vector and that causes a modulation, correction or activation of the expression of an endogenous gene or the expression of a gene introduced into the cells of the mammal or human by the vector DNA,
a) is amplified under selection pressure and is cleaved in such a manner that the said selection marker gene and the said heterologous DNA are present on separate DNA fragments,
b) the DNA fragment that contains the said heterologous DNA or both fragments are recircularized into vectors,
c) the said DNA fragments are separated before or after the recircularization,
d) the recircularized DNA fragment that contains the said heterologous DNA is isolated and
e) the recircularized DNA fragment obtained in this manner is used to produce the drug.

2. Use as claimed in claim 1, **characterized in that** the cleavage in step a) is carried out by a restriction endonuclease.

3. Use as claimed in claim 1, **characterized in that** the cleavage in step a) and the recircularization are carried out in one step by a site-specific recombination.

4. Use as claimed in claim 1 or 3, **characterized in that** a LoxP site adjoins both the 3' and 5' ends of the heterologous DNA and steps a) and b) are effected by the recombinase Cre.

5. Use as claimed in claims 1 to 3, **characterized in that** the treatment by gene therapy is carried out in the respiratory tract, in the digestive tract or on the skin surface.

6. Use as claimed in claim 5, **characterized in that** the circular vector DNA modulates or activates the expression of the endogenous gene for cystic fibrosis or codes for the cystic fibrosis gene.

7. Use as claimed in claim 6, **characterized in that** the drug is produced as an aerosol for administration in the respiratory tract.

8. Therapeutic preparation of a circular vector DNA in an amount of 30 to 90 pmol which contains a DNA sequence that is heterologous for the vector and that modulates, activates or corrects an endogenous cystic fibrosis gene in mammalian cells, or which contains a cystic fibrosis gene that can be expressed in mammalian cells, **characterized in that** this vector DNA does not contain a selection marker gene.

## Revendications

1. Utilisation d'un ADN vecteur circulaire sans gène marqueur de sélection pour produire un médicament de thérapie génique pour le traitement de mammifères ou d'êtres humains, l'ADN vecteur étant obtenu par le fait qu'un ADN vecteur qui comprend un gène marqueur de sélection et une séquence ADN hétérologue au vecteur, qui provoque une modulation, une correction ou une activation de l'expression d'un gène endogène ou l'expression d'un gène introduit dans les cellules du mammifère ou de l'être humain par l'ADN vecteur
a) est amplifié sous pression sélective et ainsi, clivé de telle façon que ledit gène marqueur de sélection et ledit ADN hétérologue se trouvent sur des fragments d'ADN séparés,
b) que le fragment d'ADN qui comprend ledit ADN hétérologue ou les deux fragments d'ADN hétérologues sont recircularisés sous forme de vecteurs,
c) que lesdits fragments d'ADN sont séparés avant et après la recircularisation,
d) que le fragment d'ADN recircularisé qui comprend ledit ADN hétérologue, est isolé et
e) que le fragment d'ADN recircularisé ainsi obtenu est utilisé pour fabriquer le médicament.

2. Utilisation selon la revendication 1, **caractérisée en ce que** le clivage de l'étape a) s'effectue par une endonucléase de restriction.

3. Utilisation selon la revendication 1, **caractérisée en ce que** le clivage de l'étape a) et la recircularisation s'effectuent en une étape par une recombinaison spécifique au site.

4. Utilisation selon la revendication 1 ou 3, **caractérisée en ce que** s'adjoint au niveau des extrémités 3' et 5' de l'ADN hétérologue, respectivement un site LoxP entraînant les étapes a) et b) par recombinase Cre.

5. Utilisation selon les revendications 1 à 3, **caractérisée en ce que** le traitement par thérapie génique s'effectue dans les voies respiratoires, dans le tractus digestif ou à la surface cutanée.

6. Utilisation selon la revendication 5, **caractérisée en ce que** l'ADN vecteur circulaire entraîne une modulation ou une activation de l'expression du gène endogène pour la fibrose kystique ou code pour le gène de la fibrose kystique.

7. Utilisation selon la revendication 6, **caractérisée en ce que** le médicament est produit sous forme d'aérosol pour l'utilisation dans les voies respiratoires.

8. Préparation thérapeutique d'un ADN vecteur circulaire en une quantité de 30 à 90 pmol qui comprend une séquence ADN hétérologue au vecteur provoquant la modulation, l'activation ou la correction d'un gène endogène de la fibrose kystique dans des cellules de mammifère ou qui comprend un gène de la fibrose kystique exprimable dans des cellules de mammifère, **caractérisée en ce que** cet ADN vecteur ne contient pas de gène marqueur de sélection.
